# EUROPÄISCHE PATENTSCHRIFT

(11) **EP 1 337 201 B1**
(45) Veröffentlichungstag und Bekanntmachung des Hinweises auf die Patenterteilung: **01.06.2005**
(21) Anmeldenummer: 01995534.3
(22) Anmeldetag: 29.11.2001
(51) Int. Cl.: A61F 2/14, A61F 9/007, B29D 11/02

(54) **HORNHAUTPROTHESE**
KERATOPROTHESIS
PROTHESE CORNEENNE

(30) Priorität: 30.11.2000 DE 10059852
(43) Veröffentlichungstag der Anmeldung: 27.08.2003
(73) Patentinhaber: Aachener Centrum für Technologietransfer in der Ophthalmologie e.V., 52070 Aachen (DE); Freundes- und Förderkreis des Institutes für Textiltechnik der RWTH Aachen e.V., 52062 Aachen (DE)
(72) Erfinder: KLEE, Doris, 52074 Aachen (DE); KINGSHOTT, Peter;, DK-Roskilde (DK); ADEMOVIC, Zahida, AC-52074 Aachen (DE); SCHRAGE, Norbert, 52070 Aachen (DE); BERNDT, Erik, 67655 Kaiserslautern (DE)
(74) Vertreter: Priesmeyer, Thomas
(86) Internationale Anmeldenummer: PCT/DE2001/004459
(87) Internationale Veröffentlichungsnummer: WO 2002/043622

(56) Entgegenhaltungen:
- EP-A- 0 469 993
- EP-A- 0 956 834
- WO-A-91/03990
- DE-A- 2 705 234
- DE-A- 19 508 922
- NL-A- 8 501 403
- US-A- 4 655 774
- US-A- 4 693 715
- US-A- 5 262 097

## Beschreibung

Die Erfindung betrifft eine Homhautprothese mit einem transparenten optischen Zentrum und mit einer dieses umgebenden Randzone, wobei die Homhautprothese nach Art einer flexiblen gewölbten Sklerallinse aus flexiblem Material ausgebildet ist und die Randzone eine kovalent gebundene, biokompatible Beschichtung zur Erzielung hoher Adherenz von Zellverbänden aufweist.

Aus der DE 195 08 922 C2 ist eine Homhautprothese mit einem klaren optischen Zentrum und mit einer dieses umgebenden Randzone bekannt, bei der die Homhautprothese nach Art einer flexiblen gewölbten Sklerallinse aus flexiblem Material ausgebildet ist, die Innenfläche des optischen Zentrums eine kovalent an das Linsenmaterial gebundene, biokompatible antiadhäsive Beschichtung zur Verminderung der Adherenz von Zellverbänden aufweist und die Randzone eine kovalent an das Linsenmaterial gebundene, biokompatible adhäsive Beschichtung zur Erzielung hoher Adherenz von Zellverbänden aufweist. Nachteilig an dieser Ausführung ist, dass diese Homhautprothese sich aufgrund verschiedener Einflüsse nicht als Dauerimplantat eignet. So können sich bei dieser Linse im Augeninnern Radikale bilden, die das Auge schädigen und die Durchsicht durch das optische Zentrum verhindern. Ein zu kurz ausgebildetes Randzonenmaterial mit ungenügenden mechanischen Eigenschaften begünstigt ein Ausleihern und somit Lockern der Prothese. Bereits einer der genannten und in der praktischen Anwendung gehäuft auftretenden Faktoren reicht aus, um die Dauerhaftigkeit einer Homhautprothese deutlich herabzusetzen.

Die Aufgabe der vorliegenden Erfindung besteht nun darin, eine Hornhautprothese zu schaffen, die dauerhaft einwächst ohne ihre guten mechanischen und optischen Eigenschaften zu verlieren.

Zur Lösung dieser Aufgabe ist erfindungsgemäß bei einer Homhautprothese der eingangs erwähnten Art vorgesehen, dass das optische Zentrum innerlich und/oder äußerlich mit UV-Absorbem versehen ist, dass die Außenkante der Randzone nach Implantation der Homhautprothese durchgehend bis hinter den Bereich der Augenmuskelansätze reicht, dass die Randzone im Bereich der Augenmuskeln zwischen Augapfel und Augenmuskeln im Bereich des Äquators fixiert ist, und dass das optische Zentrum auf der dem Auge zugewandten Seite zur Randzone durch eine umlaufende Implantationslippe begrenzt ist. Dadurch wird unter Ausschluß der in der Praxis bekannten Störfaktoren ein dauerhaftes Implantat geschaffen, das dem Träger langfristig eine ausreichende Sehfähigkeit ermöglicht.

Erfindungsgemäß kann vorgesehen sein, dass das optische Zentrum aus transparenten Polymeren und / oder Elastomeren, insbesondere Polyester, Silikon und / oder Polyethylen besteht. Dabei gilt es für den jeweiligen Einsatz das optimale Material bzw. eine Kombination mehrerer Materialien zu wählen.

Auch kann erfindungsgemäß vorgesehen sein, dass die dem Auge zugewandte Oberfläche des optischen Zentrums eine kovalent gebundene, biokompatible antiadhäsive Beschichtung zur Verminderung der Adherenz von Zellverbänden aufweist. Somit bleibt die Transparenz des optischen Zentrums erhalten.

Weiterhin kann erfindungsgemäß vorgesehen sein, dass das optische Zentrum zumindest teilweise eingefärbt und / oder bedruckt ist. Damit kann eine Pupillenzeichnung nachempfunden werden. Das operierte Auge wird somit einem gesunden Auge ähnlicher.

Auch kann erfindungsgemäß vorgesehen sein, dass die Randzone Polyvinylidenfluorid (PVDF), Polyester, Polypropylen, Polytetrafluorethylen, Polyetherketon, Polyetheretherketon, natürliche Polymere, künstliche Polymere, polymere Kunststoffe, Acrylatverbindungen, Chemiefasern, Derivate und/oder Kombinationen hieraus beinhaltet. Dadurch können zusammen mit dem PVDF für den jeweiligen Einsatz optimierte mechanische Eigenschaften erzielt werden.

Erfindungsgemäß kann zudem vorgesehen sein dass die Randzone zumindest partiell poröse Strukturen, insbesondere textile Strukturen als textiler Einwachsbereich, aufweist. Textile Einwachsbereiche ermöglichen aufgrund ihrer genau einzustellenden Porosität den Zellen ein gutes Einwachsen.

Auch kann erfindungsgemäß vorgesehen sein, die Randzone zumindest teilweise einzufärben, zu bedrucken und / oder zu besticken ist. Damit kann die natürliche Iriszeichnung nachempfunden werden

Weiterhin kann erfindungsgemäß vorgesehen sein, dass die Randzone Ausformungen zur Aufnahme von Nahtmaterial aufweist. Ein schnelles genaues Einnähen während der Operation wird somit gewährleistet.

Erfindungsgemäß kann vorgesehen sein, dass die Oberfläche der Randzone durch eine Beschichtung, insbesondere eine kovalent gebundene, biokompatible Beschichtung, bioaktiv ist.

Erfindungsgemäß kann zudem vorgesehen sein, dass die bioaktive Oberfläche der Randzone, insbesondere deren gegebenenfalls vorhandene Beschichtung, Ankermoleküle aufweist, die aus zumindest einer kovalent an die Randzone gebundenen chemischen Verbindung mit zumindest einer funktionellen Gruppe bestehen. Derartige Ankermoleküle verhindern die adsorptive unkontrollierte Anlagerung von Biomolekülen, insbesondere Proteinen.

Auch kann erfindungsgemäß vorgesehen sein, dass die bioaktive Oberfläche der Randzone, insbesondere deren gegebenenfalls vorhandene Beschichtung, mit einer funktionellen Gruppe versehene Biomoleküle aufweist, wobei die Biomoleküle über zumindest eine ihrer funktionellen Gruppen mit funktionellen Gruppen der Ankermoleküle verbunden ist. Dadurch wird eine kovalente dauerhafte Anlagerung der Biomoleküle ermöglicht, wobei Konformitätsänderungen der Biomoleküle weitestgehend verhindert werden können.

Weiterhin kann erfindungsgemäß vorgesehen sein, dass die Biomoleküle zumindest ein bioaktives Zentrum zur Ankopplung von Zellen aufweisen. Somit wird ein schnelles Anwachsen von Zellen ermöglicht. Das Implantat wird schnell in den Körper integriert.

Zudem kann erfindungsgemäß vorgesehen sein, dass die Beschichtung der Randzone mit funktionellen Gruppen versehene Spacermoleküle aufweist, wobei die Spacermoleküle zwischen Ankermolekülen und Biomolekülen angeordnet sind und ihre funktionellen Gruppen mit den funktionellen Gruppen der Ankermoleküle und Biomoleküle kovalent verbunden sind. Die Spacer fungieren als Abstandhalter, die kovalent zu bindende Biomoleküle auf Abstand von der Randzone halten. Somit werden gebundene Biomoleküle nur geringfügig in ihrer Bewegungsfreiheit eingeschränkt und können ihre natürliche Konformation bewahren.

Erfindungsgemäß können als Ankermoleküle pfropfcopolymerisierte Poly(-acrylsäure-) ketten vorgesehen sein. Als funktionelle Gruppe(n) der Ankermoleküle können Carboxylgruppen und als funktionelle Gruppe(n) der Biomoleküle Aminogruppen vorgesehen sein.

Dabei können als Biomoleküle zelladhäsionsfördemde Proteine und/oder deren Fragmente aber auch Wachstumsfaktoren, insbesondere Platelet Derived Growth Factor (PDGF), eingesetzt sein.

Weiterhin können die Biomoleküle als kleine Peptide, die eine zellbindende Domaine enthalten, insbesondere RGD-haltige Peptide von 3 bis 40 Aminosäuren umfassenden Peptidsequenzen, vorgesehen sein.

Auch kann erfindungsgemäß vorgesehen sein, dass die bioaktive Oberfläche der Randzone, insbesondere deren gegebenenfalls vorhandene Beschichtung, Beschichtung Aktivatoren für die Wundheilung beinhaltet.

Zudem kann erfindungsgemäß vorgesehen sein, dass die bioaktive Oberfläche der Randzone, insbesondere deren gegebenenfalls vorhandene Beschichtung, Reaktionsbeschleuniger für die Zellverbundbildung beinhaltet. Auch dies hilft beim schnelleren Einheilen der Homhautprothese.

Erfindungsgemäß kann auch vorgesehen sein, den Einwachsbereich als Gewirk, Geflecht, Gestrick, Gewebe, Schaum, Folie, Vlies, sonstiges poröses oder perforiertes Element oder als Kombination hiervon auszubilden. Dabei können Stapelfasern, Mono-, und/oder Multifilamente Verwendung finden.

Erfindungsgemäß kann vorgesehen sein, den Einwachsbereich zumindest partiell aus resorbierbarem Material auszubilden, wobei das resorbierte Material vom Körper durch körpereigenes Material ersetzt wird.

Weiterhin kann erfindungsgemäß vorgesehen sein, dass der Einwachsbereich über die restliche Randzone und/oder direkt mit dem optischen Zentrum kraftschlüssig verbunden ist. Selbstverständlich ist auch eine form- und/oder stoffschlüssige Verbindung möglich. Die Verbindung kann z.B. durch Vulkanisieren oder Formgießen erfolgen.

Auch kann erfindungsgemäß vorgesehen sein, dass der Einwachsbereich mit der restlichen Randzone und mit dem optischen Zentrum oder direkt mit dem optischen Zentrum vergossen, verschmolzen, verklebt, vernäht, verschweißt und/oder durch Copolymeristation verbunden ist.

Erfindungsgemäß kann vorgesehen sein, dass die antiadhäsive Beschichtung Heparin, Heparinsulfate und / oder deren Abkömmlinge umfasst. Durch den Einsatz einer PEG/PEI-Hydrogelbeschichtung für das optische Zentrum kann eine spezifische Proteinadsorption gehemmt werden. Die PEG/PEI-Hydrogelbeschichtung umfasst Polyethylenglycole und deren Abkömmlinge und Polyethylenimide und deren Abkömmlinge.

Auch kann erfindungsgemäß vorgesehen sein, dass zur Ermittlung des Augeninnendruckes Mittel zur Dehnungsmessung im Bereich der Randzone, insbesondere an dem textilartigen Einwachsbereich, vorgesehen sind. Dies kann in Form von Dehnmessstreifen erfolgen. Zudem ist vorstellbar, direkt an der Randzone elektrische Signale vom piezoaktiven Komponenten, insbesondere PVDF, abzunehmen.

Erfindungsgemäß kann vorgesehen sein, am Innendurchmesser des Einwachsbereichs eine Verstärkung und/oder ein Kragen auszubilden. In der Form, dass das textile Material der Randzone in die Implantationslippe hineingeführt wird und ca. 2 mm in das Auge hineinsteht. Dort wird diese innere textile Lippe auf die Rückseite der Hornhaut genäht und bringt somit eine Zugentlastung im Auge.

Auch kann vorgesehen sein, dass am Außendurchmesser des Einwachsbereichs zum Auginneren hin eine Verstärkung ausgebildet ist.

Weiterhin kann vorgesehen sein, dass der Einwachsbereich eine für das Einwachsen der Zellen optimierte offene Porosität und Porengrößenverteilung aufweist.

Auch kann bei der erfindungsgemäßen Homhautprothese vorgesehen sein, dass die Innen- und/oder Außenfläche des optischen Zentrums für eine dioptrische Anpassung ausgestaltet sind.

Zudem kann bei einem Verfahren zur Erzeugung einer kovalent gebundenen, biokompatiblen zelladhäsiven Beschichtung auf der Randzone einer Homhautprothese vorgesehen sein, dass auf der Randzone funktionelle Gruppen aufweisende Ankermoleküle gebildet werden, indem bei einer Plasmazündung mit mikrowelleninduzierten Niederdruckplasmen durch Inertgas radikalische Gruppen erzeugt werden, welche nach der Belüftung mit Luftsauerstoff zu Peroxyradikalen und schließlich zu Hydroperoxiden abreagieren, die anschließend durch thermische und/oder photochemische Spaltung freie Radikale bilden, über die Monomere pfropfcopolymerisiert werden.mit Luftsauerstoff zu Peroxyradikalen und schließlich zu Hydroperoxiden abreagieren, die anschließend durch thermische und/oder photochemische Spaltung freie Radikale bilden, über die Monomere pfropfcopolymerisiert werden.

Auch kann erfindungsgemäß vorgesehen sein, an die funktionelle(n) Gruppe(n) der Ankermoleküle kovalent Biomoleküle über deren funktionelle Gruppe(n) zu binden. Dadurch wird das Einwachsen der Prothese in das Auge ermöglicht.

Weiterhin kann erfindungsgemäß vorgesehen sein, dass überschüssige, nicht kovalent gebundene Homopolymere im Anschluß an die Pfropfcopolymerisation entfernt werden. Ungewollte Reaktionen dieser Homopolymere werden somit vermieden.

Auch kann erfindungsgemäß vorgesehen sein, als Plasmagas Argon und/oder N₂ zu verwenden.

Erfindungsgemäß kann auch vorgesehen sein, dass zur photochemischen Spaltung eine thermische und/oder UV-induzierte Spaltung mittels Excimerstrahler erfolgt. Im Gegensatz zu Quecksilber-Hochdruck- und Niederdrucklampen ermöglichen Excimerstrahler die Emission inkohärenter schmalbandiger UV-Strahlung.

Weiterhin kann erfindungsgemäß vorgesehen sein, als zu pfropfendes Monomer Vinylmonomere, insbesondere Acrylsäure (AAc), und/oder Macromonomere, insbesondere Polyethylenglykolmethacrylate (PEGMA), zur Bildung einer hochhydrophilen Hydrogelschicht zu verwenden.

Schließlich kann erfindungsgemäß vorgesehen sein, ein PEGMA mit Poly(ethylen)kettenlängen von 4 bis 100 zur Bildung einer hochhydrophilen Hydrogelschicht einzusetzen. Die Hydrogelschicht verhindert die unerwünschte Adsorption von Proteinen. Die Schicht ermöglicht zudem die Freisetzung von Wirkstoffen, z.B. Antibiotika, über einen längeren Zeitraum. Dies hilft Infektionen zu verhindern.

Die Erfindung soll nachstehend anhand zweier Ausführungsbeispiele näher erläutert werden. In den zugehörigen Zeichnungen zeigen:
- Fig. 1: eine schematische Aufsicht auf eine erfindungsgemäße Homhautprothese,
- Fig. 2: einen schematischen Schnitt durch ein Auge mit implantierter erfindungsgemäßer Homhautprothese, und
- Fig. 3: einen schematischen Schnitt durch eine erfindungsgemäße Homhautprothese mit Mitteln zur Druckmessung.

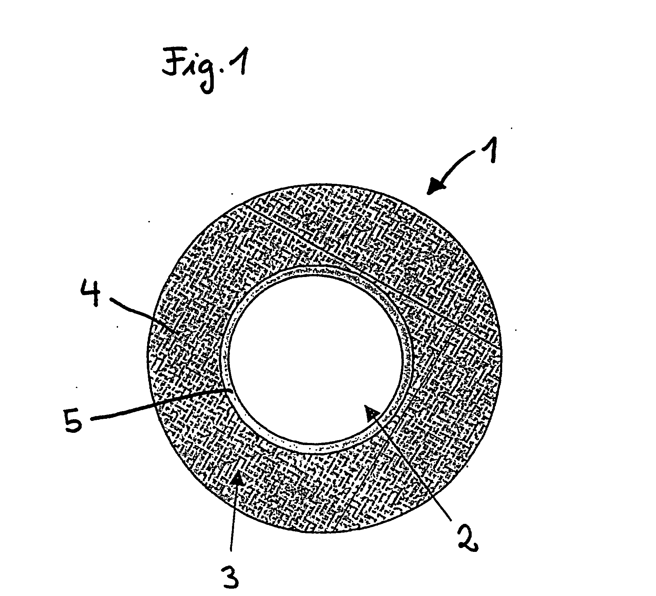

In Figur 1 ist eine kreisförmige gewölbte Homhautprothese 1 mit einem kreisförmigen klaren optischen Zentrum 2 und einer ringförmigen Randzone 3, die durchgehend als textilartiger Einwachsbereich 4 ausgebildet ist, dargestellt. Das optische Zentrum 2 hat üblicherweise einen Durchmesser von 5,0 bis 9,0 mm und die ringförmige Randzone eine Erstreckung von 3 bis 17 mm zwischen ihrem Außen- und Innendurchmesser. Das optische Zentrum 2 ist auf der dem Auge zuzuwendenden Seite zur Randzone 3 hin durch eine umlaufende Implantationslippe 5 begrenzt. Der Bereich zwischen optischem Zentrum 2 und Randzone 3, der von der Implantationslippe 5 eingenommen wird, hat eine Breite von 0,2 bis 0,6 mm. Durch die Vergrößerung der Implantationszone auf die Sklera zwischen die Sklera und die Tenonkapsel bis hinter den Bereich der Augenmuskelansätze reichend wird eine flächige Implantationszone hergestellt, die eine hohe mechanische Last aufnehmen kann.

Das optische Zentrum 2 kann aus weichem transparentem Material, z.B. transparenten Polymeren, transparenten Elastomeren (Polyester, Silikon, Polyethylen) oder einfärbbaren Polymeren bestehen und mit einer zellabweisenden Beschichtung versehen sein. Mittels einzufärbender oder zu bestickender Polymere kann eine künstliche Iris erzeugt werden. Die Wölbung der Außen- und Innenfläche des optischen Zentrums kann für eine dioptrische Anpassung gezielt gewählt werden. Durch den Einsatz eines UV-Filters kann verhindert werden, dass UV-Strahlung durch das Implantat in das Auge dringen kann und dann Radikale gebildet werden, welche eine intraokulare Entzündung bewirken und die Netzhaut schädigen. Dafür kann die Oberfläche des optischen Zentrums mittels UV-Absorbem beschichtet sein oder aber in das optische Zentrum eingebrachte Beimengungen von UV-Absorbem vorgesehen sein. Eine kovalent gebundene biokompatible antiadhäsive Beschichtung kann die Verminderung der Adherenz von Zellverbänden bewirken. Das optische Zentrum 2 ist den mechanischen Eigenschaften des umgebenden und zu ersetzenden körpereigenen Materials angepasst, wodurch Mikrotraumata im Implantatlager verhindert werden und damit ein besseres Einheilen gewährleistet wird.

Für den textilen Einwachsbereich 4 können glatte oder gekräuselte Stapelfasern, Monound Multifilamente und Kombinationen hieraus in Form von Gewirken Verwendung finden. Auch Schäume und/oder perforierte Folien können eingesetzt werden. Dabei ist der Einwachsbereich 4 den mechanischen Eigenschaften des umgebenden und zu ersetzenden Gewebes angepasst und kann zumindest teilweise aus resorbierbaren Materialien bestehen. Ein optimierte durchschnittliche Porengröße des textilen Einwachsbereichs 4 unterstützt das Einwachsen von Zellen, indem Zellverbände in die Poren des Einwachsbereiches 4 hinein emigrieren und dort eine feste Matrix zur Einheilung ausbilden können. Hierzu kann z.B. eine gerichtete Porenstruktur oder Perforation gewählt werden. Die Porengröße kann durch die Wahl des Materials, der Verarbeitung und/oder weiterer Nachbearbeitungsschritte, wie z.B. Kalandrier-, Schrumpf- und/oder Thermofixierungsprozesse eingestellt werden. Zudem können durch ein geeignetes Stick- oder Nähverfahren dem Einwachsbereich 4 eine optimierte Porengröße, mechanische Eigenschaften und Färbung zugewiesen werden. Die Färbung kann gegebenenfalls auch durch die Verwendung verschiedener Materialien bei der Herstellung der Textilstruktur erzielt werden. Gewirke weisen neben dem Vorteil einstellbarer Dehnungseigenschaften auch eine gute Schnittfähigkeit auf. Daher kann neben in der Endform hergestellten Einwachsbereichen 4 auch konfektionierbare Ware zum individuellen Zuschneiden gewählt werden.

Idealerweise wird für die Randzone 3 und insbesondere deren Einwachsbereich 4 ein PVDF verwendet, das mit Acryläure (AAc) als Monomer zur Erzeugung einer hydrophilen carboxylgruppenhaltigen Oberfläche gepfropft wurde. Ein derart oberflächenmodifiziertes PVDF ist äußerst biokompatibel. Als Beschichtung sind rekombinante Zellhaftungsproteine, d.h. molekularbiologisch hergestellte Eiweiße des Körpers, vorstellbar. Diese Zellhaftungsproteine signalisieren den Zellen als Botenstoffe gute Haftung, bzw. wirken selber als Haftungsmoleküle. Kovalent an die Randzone 3 gebundene Proteine oder atterungsgeschützte Proteinfragmente können eine Proliferation, Migration und Narbenbildung der einwachsenden Zellverbände herstellen und/oder eine periimplantäre Fibrose reduzieren. Die Beschichtung kann zudem Mittel zum Fördern der Wundheilung und zur Herstellung einer bindegewebigen Einheilung beinhalten. Derartige Beschichtungen können direkt oder über Spacer mit der Oberfläche der Randzone kovalent verbunden sein.

Der Einwachsbereich 4 kann mit der übrigen Randzone über einen Einschmelzprozess bzw. ein "Einvulkanisieren" verbunden sein. Dies ist eine Art modifiziertes Kleben ,wie man es auch beim "Eingießen" von Textil und Stahl in Autoreifen benutzt. Das optische Zentrum 2 und die Randzone 3 können z.B. miteinander verklebt, verschweißt oder copolymerisiert sein. Es wird somit zwischen Einwachsbereich und optischem Zentrum ein inniger Verbund erreicht, welcher langfristig belastungsstabil ist und nur geringe Unterschiede zu den mechanischen Eigenschaften des zu ersetzenden Augenbereichs aufweist

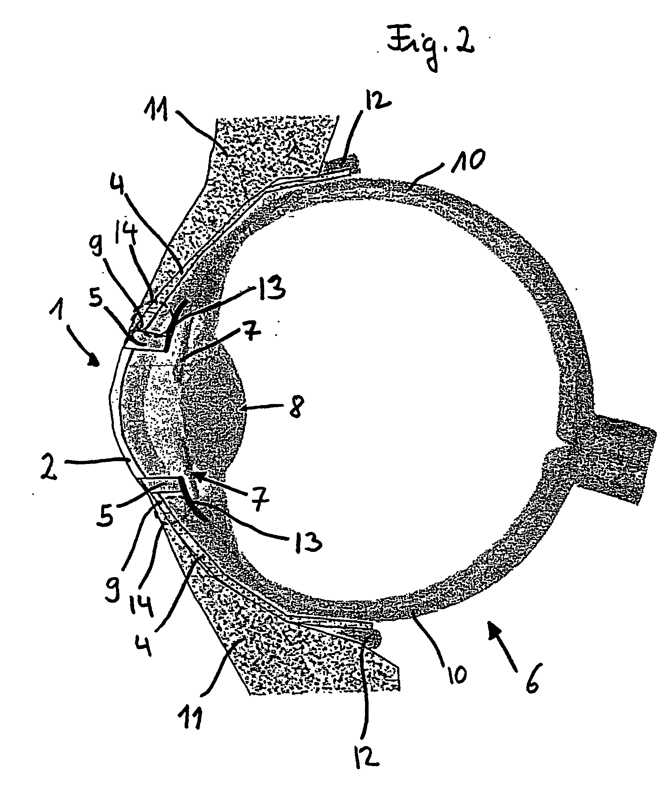

Fig. 2 zeigt eine in ein Auge 6 implantierte Hornhautprothese 1. Diese besteht aus einem weichen optischen Zentrum 2, das vor der Iris 7 und Augenlinse 8, die zur Verhinderung von retroprosthetischen Membranen optional entfernt werden könnte, angeordnet ist. Das optische Zentrum 2 hat eine Dicke von 0,2 bis 0,7 mm und ist mit einem UV-Absorber zur Erhaltung der Klarheit versehen. Eine antiadhäsive Beschichtung verhindert u.a. die Anlagerung von Radikalen und hilft somit die Transparenz des optischen Zentrums zu erhalten. Das optische Zentrum 2 ist auf der dem Auge 6 zugewandten Seite zur Randzone 3 hin durch eine umlaufende Implantationslippe 5 begrenzt, die in einen Defekt versenkt wird und somit das Auge 6 am Ort der Hornhautentnahme verschließt. Dies führt zu einer chirurgischen Situation, die später eine Wiederherstellung des vorderen Augenabschnittes durch Transplantation ermöglicht. Die Randzone 3 ist partiell als textiler Einwachsbereich 4 aus PVDF ausgebildet und in einem Übergangsbereich 9 mit dem optischen Zentrum 2 durch Vulkanisation verbunden. Der textile Einwachsbereich 4 ist zwischen der Sklera 10 und einer Bindegewebshülle (Schleimhautplastik respektive Tenonplastik) 11 angeordnet Die Bindegewebshülle 11 ist auf den textilen Einwachsbereich 4 aufgewachsen und in den Porenraum des Einwachsbereiches 4 eingewachsen. Der Einwachsbereich 4 erstreckt sich bis hinter die Augenmuskelansätze 12 und ist im Bereich unter den Augenmuskeln durchgezogen und unter anderem seitlich der Augenmuskeln und auf der Sklera mit Steppnähten z.B. durch Nähte fixiert. Zudem befindet sich an dem dem Augeninnem zugewandten Ende der Implantationslippe 5 eine überwiegend parallel zur Randzone 3 verlaufende innere Lippe 13 als auf die Rückseite des Absetzungstumpfes der Hornhaut reichende Struktur des Einwachsbereiches, die mit einer skizziert dargestellten Naht 14 mit dem Homhautstroma verbunden ist. Die Erstreckung der inneren Lippe 13 zwischen Innenund Außendurchmesser beträgt ca. 3 bis 4 mm.

Selbstverständlich sind auch andere und zusätzliche Fixationsmöglichkeiten, z.B. in Form von Cerclagen, vorstellbar. Auch können Entwässerungskanäle vorgesehen sein. Durch eine geschickte Auswahl der Texturierung der Randzone sowie eine Einstellung von Kombinationen von nicht resorbierbaren und resorbierbaren Materialien kann eine spezifische Einheilung induziert werden. Damit ist die Heilungsphase steuerbar und eine vorhersagbare Einheilung einzustellen. Durch die Implantation von zellproliferationsfördemden Mediatoren (PDGF) oder zelladhäsionsfördernden Proteinen oder deren Fragmenten lassen sich diese Heilungsprozesse induzieren und unterhalten.

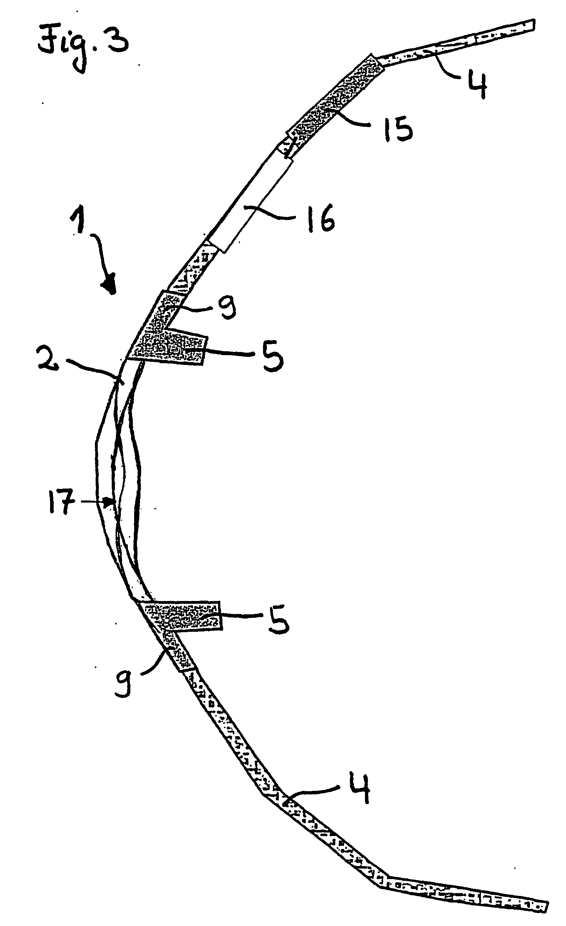

In Figur 3 ist eine erfindungsgemäße weiche Homhautprothese 1 mit einem textilen Einwachsbereich 4 aus PVDF und mit Mitteln zur intraokularen Druckmessung dargestellt. Im Bereich der Implantationslippe 5 sind die textile Struktur des Einwachsbereiches und die elastornere Struktur der übrigen Randzone 3 miteinander verwoben bzw. vulkanisiert. Die Mittel zur Innendruckmessung umfassen einen Sender 15 für ausgewertete Augendrucksignale. Am zugsensiblen Bereich des PVDF sind hier nicht dargestellte Elektroden vorgesehen, die Piezoströme abnehmen und diese an den Transducer 16 und den Sender 15 weiterleiten. Auch ist der Einsatz von Dehnmessstreifen vorstellbar. Wird nun auf das optische Zentrum eine deformierende Kraft ausgeübt, so führt dies z.B. zu einer Applanation oder Indentation 17. Ebenso kann durch die Kammerwasserproduktion das intraokuläre Volumen des Auges 6 ansteigen. Die resultierende druckabhängige Verformung der Befestigung der Keratoprothese im Bereich der Sklera kann dann mittels mikronisierter Telemetrie erfasst werden. Über eine hier nicht dargestellte Mikropumpentechnik kann dann der Augeninnendruck geregelt werden.

### Bezugszeichenliste

- 1.: Hornhautprothese
- 2.: Optisches Zentrum
- 3.: Randzone
- 4.: Einwachsbereich
- 5.: Implantationslippe
- 6.: Auge
- 7.: Iris
- 8.: Augenlinse
- 9.: Übergangsbereich
- 10.: Sklera
- 11.: Bindegewebshülle
- 12.: Augenmuskeln
- 13.: Innere Lippe
- 14.: Naht
- 15.: Sender
- 16.: Transducer
- 17.: Applanation/Indentation

## Patentansprüche

1. Hornhautprothese mit einem transparenten optischen Zentrum (2) und mit einer dieses umgebenden Randzone (3), wobei die Homhautprothese nach Art einer flexiblen gewölbten Skierallinse aus flexiblem Material ausgebildet ist und die Randzone (3) mit einer bioaktiven Oberfläche zur Erzielung hoher Adherenz von Zellverbänden ausgestattet ist,
**dadurch gekennzeichnet,**
- **dass** das optische Zentrum (2) innerlich und/oder äußerlich mit UV-Absorbem versehen ist,
- **dass** die Außenkante der Randzone (3,4) nach Implantation der Homhautprothese durchgehend bis hinter den Bereich der Augenmuskelansätze reicht,
- **dass** die Randzone (3) im Bereich der Augenmuskeln (12) zwischen Augapfel und Augenmuskeln (12) im Bereich des Äquators fixiert werden kann, und
- **dass** das optische Zentrum (2) auf der dem Auge (6) zugewandten Seite zur Randzone (3) durch eine umlaufende Implantationslippe (5) begrenzt ist.

2. Hornhautprothese nach Anspruch 1, **dadurch gekennzeichnet, dass** das optische Zentrum (2) aus transparenten Polymeren und / oder Elastomeren, insbesondere Polyester, Silikon und / oder Polyethylen besteht.

3. Hornhautprothese nach Anspruch 1 oder 2, **dadurch gekennzeichnet, dass** die dem Auge (6) zugewandte Oberfläche des optischen Zentrums (2) eine kovalent gebundene, biokompatible antiadhäsive Beschichtung zur Verminderung der Adherenz von Zellverbänden aufweist.

4. Hornhautprothese nach einem der vorhergehenden Ansprüche, **dadurch gekennzeichnet, dass** das optische Zentrum (2) zumindest teilweise eingefärbt und / oder bedruckt ist.

5. Hornhautprothese nach einem der vorhergehenden Ansprüche, **dadurch gekennzeichnet, dass** die Randzone (3) Polyvinylidenfluorid (PVDF), Polyester, Polypropylen, Polytetrafluorethylen, Polyetherketon, Polyetheretherketon, natürliche Polymere, künstliche Polymere, polymere Kunststoffe, Acrylatverbindungen, Chemiefasern, Derivate und/oder Kombinationen hieraus beinhaltet.

6. Hornhautprothese nach einem der vorhergehenden Ansprüche, **dadurch gekennzeichnet, dass** die Randzone (3) zumindest partiell poröse Strukturen, insbesondere textile Strukturen als textiler Einwachsbereich (4), aufweist.

7. Hornhautprothese nach einem der vorhergehenden Ansprüche, **dadurch gekennzeichnet, dass** die Randzone (3) zumindest teilweise eingefärbt, bedruckt und / oder bestickt ist.

8. Hornhautprothese nach einem der vorhergehenden Ansprüche, **dadurch gekennzeichnet, dass** die Randzone (3) Ausformungen zur Aufnahme von Nahtmaterial aufweist.

9. Hornhautprothese nach einem der vorhergehenden Ansprüche, **dadurch gekennzeichnet, dass** die Oberfläche der Randzone durch eine Beschichtung, insbesondere eine kovalent gebundene, biokompatible Beschichtung, bioaktiv ist.

10. Hornhautprothese nach einem der vorhergehenden Ansprüche, **dadurch gekennzeichnet, dass** die bioaktive Oberfläche der Randzone (1), insbesondere deren gegebenenfalls vorhandene Beschichtung, Ankermoleküle aufweist, die aus zumindest einer kovalent an die Randzone (3) gebundenen chemischen Verbindung mit zumindest einer funktionellen Gruppe bestehen.

11. Hornhautprothese nach Anspruch 10, **dadurch gekennzeichnet, dass** die bioaktive Oberfläche der Randzone (3), insbesondere deren gegebenenfalls vorhandene Beschichtung, mit einer funktionellen Gruppe versehene Biomoleküle aufweist, wobei die Biomoleküle über zumindest eine ihrer funktionellen Gruppen mit funktionellen Gruppen der Ankermoleküle verbunden ist.

12. Hornhautprothese nach Anspruch 11, **dadurch gekennzeichnet, dass** die Biomoleküle zumindest ein bioaktives Zentrum zur Ankopplung von Zellen aufweisen.

13. Hornhautprothese nach Anspruch 11 oder 12, **dadurch gekennzeichnet, dass** die bioaktive Oberfläche der Randzone (3), insbesondere deren gegebenenfalls vorhandene Beschichtung, mit funktionellen Gruppen versehene Spacermoleküle aufweist, wobei die Spacermoleküle zwischen Ankermolekülen und Biomolekülen angeordnet sind und ihre funktionellen Gruppen mit den funktionellen Gruppen der Ankermoleküle und Biomoleküle kovalent verbunden sind.

14. Hornhautprothese nach einem der Ansprüche 10 bis 13, **dadurch gekennzeichnet, dass** als Ankermoleküle pfropfcopolymerisierte Poly(-acrylsäure-) ketten vorgesehen sind.

15. Hornhautprothese nach einem der Ansprüche 10 bis 14, **dadurch gekennzeichnet, dass** als funktionelle Gruppe(n) der Ankermoleküle Carboxylgruppen vorgesehen sind.

16. Hornhautprothese nach einem der Ansprüche 11 bis 15, **dadurch gekennzeichnet, dass** als funktionelle Gruppe(n) der Biomoleküle Aminogruppen vorgesehen sind.

17. Hornhautprothese nach einem der Ansprüche 11 bis 16, **dadurch gekennzeichnet, dass** als Biomoleküle zelladhäsionsfördernde Proteine und/oder deren Fragmente vorgesehen sind.

18. Hornhautprothese nach einem der Ansprüche 11 bis 17, **dadurch gekennzeichnet, dass** als Biomoleküle Wachstumsfaktoren, insbesondere Platelet Derived Growth Factor (PDGF), vorgesehen sind.

19. Hornhautprothese nach einem der Ansprüche 11 bis 18, **dadurch gekennzeichnet, dass** als Biomoleküle kleine Peptide, die eine zellbindende Domaine enthalten, insbesondere RGD-haltige Peptide von 3 bis 40 Aminosäuren umfassenden Peptidsequenzen, vorgesehen sind.

20. Hornhautprothese nach einem der vorhergehenden Ansprüche, **dadurch gekennzeichnet, dass** die bioaktive Oberfläche der Randzone (3), insbesondere deren gegebenenfalls vorhandene Beschichtung, Beschichtung Aktivatoren für die Wundheilung beinhaltet.

21. Hornhautprothese nach einem der vorhergehenden Ansprüche, **dadurch gekennzeichnet, dass** die bioaktive Oberfläche der Randzone (3), insbesondere deren gegebenenfalls vorhandene Beschichtung, Reaktionsbeschleuniger für die Zellverbundbildung beinhaltet.

22. Hornhautprothese nach einem der Ansprüche 6 bis 21, **dadurch gekennzeichnet, dass** der Einwachsbereich (4) als Gewirk, Geflecht, Gestrick, Gewebe, Schaum, Folie, Vlies, sonstiges poröses oder perforiertes Element oder als Kombination hiervon ausgebildet ist.

23. Hornhautprothese nach einem der Ansprüche 6 bis 22, **dadurch gekennzeichnet, dass** der Einwachsbereich (4) zumindest partiell aus resorbierbarem Material ausgebildet ist.

24. Hornhautprothese nach einem der Ansprüche 6 bis 23, **dadurch gekennzeichnet, dass** der Einwachsbereich (4) über die restliche Randzone und/oder direkt mit dem optischen Zentrum (2) kraftschlüssig verbunden ist.

25. Hornhautprothese nach einem der Ansprüche 6 bis 24, **dadurch gekennzeichnet, dass** der Einwachsbereich (4) mit der restlichen Randzone und mit dem optischen Zentrum (2) oder direkt mit dem optischen Zentrum (2) vergossen, verschmolzen, verklebt, vernäht, verschweißt und/oder durch Copolymeristation verbunden ist.

26. Hornhautprothese nach einem der Ansprüche 3 bis 25, **dadurch gekennzeichnet, dass** die antiadhäsive Beschichtung für das optische Zentrum (2) Heparin, Heparinsulfate und / oder deren Abkömmlinge umfasst.

27. Hornhautprothese nach einem der vorhergehenden Ansprüche, **dadurch gekennzeichnet, dass** zur Ermittlung des Augeninnendruckes Mittel zur Dehnungsmessung im Bereich der Randzone (3), insbesondere an dem textilartigen Einwachsbereich (4), vorgesehen sind.

28. Hornhautprothese nach einem der Ansprüche 6 bis 27, **dadurch gekennzeichnet, dass** am Innendurchmesser des Einwachsbereichs (4) eine Verstärkung und/oder ein Kragen ausgebildet ist.

29. Hornhautprothese nach einem der Ansprüche 6 bis 28, **dadurch gekennzeichnet, dass** am Außendurchmesser des Einwachsbereichs (4) zum Auginneren hin eine Verstärkung ausgebildet ist.

30. Hornhautprothese nach einem der Ansprüche 6 bis 29, **dadurch gekennzeichnet, dass** der Einwachsbereich (4) eine für das Einwachsen der Zellen optimierte offene Porosität und Porengrößenverteilung aufweist.

31. Hornhautprothese nach einem der vorhergehenden Ansprüche, **dadurch gekennzeichnet, dass** die Innen- und/oder Außenfläche des optischen Zentrums (2) für eine dioptrische Anpassung ausgestaltet sind.

32. Verfahren zur Erzeugung einer kovalent gebundenen, biokompatiblen zelladhäsiven Beschichtung auf der Randzone (3) einer Hornhautprothese nach einem der vorangehenden Ansprüche, **dadurch gekennzeichnet, dass** auf der Randzone (3) funktionelle Gruppen aufweisende Ankermoleküle gebildet werden, indem bei einer Plasmazündung mit mikrowelleninduzierten Niederdruckplasmen durch Inertgas radikalische Gruppen erzeugt werden, welche nach der Belüftung mit Luftsauerstoff zu Peroxyradikalen und schließlich zu Hydroperoxiden abreagieren, die anschließend durch thermische und/oder photochemische Spaltung freie Radikale bilden, über die Monomere pfropfcopolymerisiert werden.

33. Verfahren nach Anspruch 32, **dadurch gekennzeichnet, dass** an die funktionelle(n) Gruppe(n) der Ankermoleküle kovalent Biomoleküle über deren funktionelle Gruppe(n) gebunden werden.

34. Verfahren nach Anspruch 32 oder 33, **dadurch gekennzeichnet, dass** überschüssige, nicht kovalent gebundene Homopolymere im Anschluß an die Pfropfcopolymerisation entfernt werden.

35. Verfahren nach einem der Ansprüche 32 bis 34, **dadurch gekennzeichnet, dass** als Plasmagas Argon und/oder N₂ verwendet wird.

36. Verfahren nach einem der Ansprüche 32 bis 35, **dadurch gekennzeichnet, dass** zur photochemischen Spaltung eine thermische und/oder UV-induzierte Spaltung mittels Excimerstrahler erfolgt.

37. Verfahren nach einem der Ansprüche 32 bis 36, **dadurch gekennzeichnet, dass** als zu pfropfendes Monomer Vinylmonomere, insbesondere Acrylsäure (AAc), und/oder Macromonomere, insbesondere Polyethylenglykolmethacrylate (PEGMA), zur Bildung einer hochhydrophilen Hydrogelschicht verwendet werden.

38. Verfahren nach Anspruch 37, **dadurch gekennzeichnet, dass** ein PEGMA mit Poly(ethylen)kettenlängen von 4 bis 100 zur Bildung einer hochhydrophilen Hydrogelschicht eingesetzt wird.

## Claims

1. Corneal prosthesis with a transparent optic center (2) and a peripheral zone (3) which surrounds it, whereby the corneal prosthesis is shaped like a flexible, convex scleral lens from flexible material and the peripheral zone (3) is equipped with a bioactive surface for obtaining high adherence of cell clusters,
**characterized by**,
• optic center (2) being equipped internally and/or externally with UV-absorbers,
• after implantation of the corneal prosthesis, the outer edge of the peripheral zone (3, 4) extending continuously to behind the area of the eye muscles inserts,
• the peripheral zone (3) in the area of the eye muscles (12) capable of being fixed between the eyeball and eye muscle in the area of the equator, and
• the optic center (2) being limited by a surrounding implantation lip (5) on the side of the peripheral zone (3) facing the eye.

2. Corneal prosthesis as claimed in claim 1, **characterized by** the optic center (2) consisting of of transparent polymers, silicone and/or polyethylene.

3. Corneal prosthesis as claimed in claim 1 or 2, **characterized by** the surface of the optic center (2) which faces the eye (6) showing a covalently bound, biocompatible antiadhesive coating for reducing the adherence of cell clusters.

4. Corneal prosthesis as claimed in any of the previous claims, **characterized by** the optic center (2) being at least partially dyed and/or printed.

5. Corneal prosthesis as claimed in any of the previous claims, **characterized by** the peripheral zone (3) containing polyvinylidene fluoride (PVDF), polyester, polypropylene, polytetrafluoroethylene, polyether ketone, polyether-ether ketone, natural polymers, artificial polymers, polymeric plastics, acrylic compounds, chemical fibers, derivatives and/or combinations thereof.

6. Corneal prosthesis as claimed in any of the previous claims, **characterized by** the peripheral zone (3) exhibiting at least partially porous structures, in particular, textile structures as textile incorporation area (4).

7. Corneal prosthesis as claimed in any of the previous claims, **characterized by** the peripheral zone (3) being at least partially dyed, printed and/or embroidered.

8. Corneal prosthesis as claimed in any of the previous claims, **characterized by** the peripheral zone (3) showing forms for taking up suture material.

9. Corneal prosthesis as claimed in any of the previous claims, **characterized by** the surface of the peripheral zone being bioactive through a coating, in particular, a covalently bound, biocompatible coating.

10. Corneal prosthesis as claimed in any of the previous claims, **characterized by** the bioactive surface of the peripheral zone (3), in particular its possibly existing coating, exhibiting anchor molecules which consist of at least one chemical compound with at least one functional group bound covalently to the peripheral zone (3).

11. Corneal prosthesis as claimed in claim 10, **characterized by** the bioactive surface of the peripheral zone (3), in particular its possible existing coating, exhibiting biomolecules equipped with a functional group, whereby the biomolecules are linked via at least one of their functional groups with functional groups of the anchor molecules.

12. Corneal prosthesis as claimed in claim 11, **characterized by** the biomolecules displaying at least one bioactive center for docking cells.

13. Corneal prosthesis as claimed in claim 11 or 12 , **characterized by** the bioactive surface of peripheral zone (3), in particular its possibly existing coating, exhibiting spacer molecules equipped with functional groups, whereby the spacer molecules are positioned between the anchor molecules and the biomolecules and their functional groups are covalently bound with the functional groups of the anchor molecules and biomolecules.

14. Corneal prosthesis as claimed in any of the claims 10 through 13, **characterized by** graft copolymerized polyacrylic acid chains being intended as the anchor molecules.

15. Corneal prosthesis as claimed in any of the claims 10 through 14, **characterized by** carboxyl groups being intended as functional group(s) of the anchor molecules.

16. Corneal prosthesis as claimed in any of the claims 11 through 15, **characterized by** amino groups being intended as functional group(s) of the biomolecules.

17. Corneal prosthesis as claimed in any of the claims 11 through 16, **characterized by** cell adhesion-promoting proteins and/or their fragments being intended as biomolecules.

18. Corneal prosthesis as claimed in any of the claims 11 through 17, **characterized by** growth factors, especially Platelet Derived Growth Factor (PDGF), being intended as biomolecules.

19. Corneal prosthesis as claimed in any of the claims 11 through 18, **characterized by** small peptides which contain a cell-binding domain, in particular RGD-containing peptides of peptide sequences comprising from 3 up to 40 amino acids, being intended as biomolecules.

20. Corneal prosthesis as claimed in any of the previous claims, **characterized by** the bioactive surface of peripheral zone (3), in particular its possibly existing coating, containing coating activators for the wound healing.

21. Corneal prosthesis as claimed in any of the previous claims, **characterized by** the bioactive surface of the peripheral zone (3), in particular its possibly existing coating, containing reaction accelerators for the cell attachment.

22. Corneal prosthesis as claimed in any of the claims 6 though 21, **characterized by** the incorporation area (4) being formed as a web, mesh, knit, fabric, foam, film, fleece, other porous or perforated element or as a combination thereof.

23. Corneal prosthesis as claimed as in any of the claims 6 through 22, **characterized by** the incorporation area (4) being formed at least partially from resorbable material.

24. Corneal prosthesis as claimed in any of the claims 6 through 23, **characterized by** the incorporation area (4) being connected by a non-positive connection (whereby the force is transmitted by frictional contact) with the remaining peripheral zone and/or directly with the optic center (2).

25. Corneal prosthesis as claimed in any of the claims 6 through 24, **characterized by** the incorporation area (4) being casted, melted, glued, sutured, welded and/or linked by copolymerization with the remaining peripheral zone and with the optic center (2) or directly with the optic center (2).

26. Corneal prosthesis as claimed in any of the claims 3 through 25, **characterized by** the antiadhesive coating for the optic center (2) comprising heparin, heparin sulfate and/or its derivatives.

27. Corneal prosthesis as claimed in any of the previous claims, **characterized by** means for stretch measurements being intended for establishing the intraocular pressure in the area of the peripheral zone (3), in particular at the textile-like incorporation area (4).

28. Corneal prosthesis as claimed in any of the claims 6 through 27, **characterized by** a reinforcement and/or a collar being formed at the inner diameter of the incorporation area (4).

29. Corneal prosthesis as claimed in any of the claims 6 through 28, **characterized by** a reinforcement being formed at the outer diameter of the incorporation area (4) facing the interior of the eye.

30. Corneal prosthesis as claimed in any of the claims 6 through 29, **characterized by** the incorporation area (4) showing an optimized open porosity and pore-site distribution for the cells to grow in.

31. Corneal prosthesis as claimed in any of the previous claims, **characterized by** the inner and outer area of the optic center (2) being equipped for a dioptric fitting.

32. Methods for producing a covalently bound, biocompatible cell-adhesive coating on the peripheral zone (3) of a corneal prosthesis as claimed as in any of the previous claims, **characterized by** formation of anchor molecules with functional groups on the peripheral zone, in which radical groups are produced by inert gas during a plasma ignition with microwave-induced low-pressure plasmas. After aeration, these radical groups react with atmospheric oxygen to peroxy radicals and, finally, to hydroperoxides which, subsequently, as the result of thermal and/or photochemical cleavage, create free radicals which cause monomers to graft-copolymerize.

33. Methods as claimed in claim 32, **characterized by** biomolecules being covalently bound via their functional group(s) to the functional group(s) of the anchor molecules.

34. Methods as claimed in claim 32 or 33, **characterized by** excess, non-covalently bound homopolymers being removed after the graft copolymerization.

35. Methods as claimed in any of the claims 32 through 34, **characterized by** argon and/or N₂ being applied as plasma gas.

36. Methods as claimed in any of the claims 32 through 35, **characterized by** a thermal and/or UV-induced cleavage taking place by means of an excimer radiator for photochemical cleavage.

37. Methods as claimed in any of the claims 32 through 36, **characterized by** vinyl monomers, especially acrylic acid (AAc), and/or macromonomers, in particular polyethylene glycol methacrylate (PEGMA), being used as the graft monomer for forming a highly hydrophilic hydrogel layer.

38. Methods as claimed in 37, **characterized by** a PEGMA with polyethylene chain lengths of 4 up to 100 carbon atoms being applied for forming a highly hydrophilic hydrogel layer.

## Revendications

1. Prothèse cornéenne avec un centre optique transparent (2) et une zone périphérique (3) entourant ce dernier, telle que la prothèse cornéenne soit formée à la manière d'une lentille sclérale flexible et voûtée d'un matériau flexible et que la zone périphérique (3) soit dotée d'une surface bioactive pour l'obtention d'une adhérence élevée de structures cellulaires,
avec les caractéristiques particulières
- que le centre optique (2) est doté à l'intérieur et/ou à l'extérieur d'absorbeurs UV,
- que la lisière extérieure de la zone périphérique (3,4) s'étende après implantation de la prothèse cornéenne continûment jusque derrière la région des racines des muscles oculaires,
- que la zone périphérique (3) dans la région des muscles oculaires (12) entre globe et muscles oculaires (12) peut être fixée dans la région de l'équateur, et
- que le centre optique (2) est bordé du côté de l'oeil (6) vers la zone périphérique (3) par une lèvre d'implantation circulaire.

2. Prothèse cornéenne selon l'exigence 1, avec la caractéristique particulière que le centre optique (2) est composé de polymères transparents et/ou élastomères, en particulier du polyester, du silicone et/ou du polyéthylène.

3. Prothèse cornéenne selon les exigences 1 ou 2, avec la caractéristique particulière que surface du centre optique (2) du côté de l'oeil comporte un revêtement lié par covalence, biocompatible et antiadhésive pour la réduction de l'adhérence des structures cellulaires.

4. Prothèse cornéenne selon une des exigences précédentes, avec la caractéristique particulière que le centre optique est, au moins partiellement, coloré et/ou imprimé.

5. Prothèse cornéenne selon une des exigences précédentes, avec la caractéristique particulière que la zone périphérique (3) contient des polyvinylidènes fluorides (PVDF), du polyester, du polypropylène, du polytetra-fluoro-éthylène, du polyéther-cétone, du polyéther-éther-cétone, des polymères naturels, des polymères artificiels, des composés d'acrylate, des fibres chimiques, des dérivés et/ou composés de ces derniers.

6. Prothèse cornéenne selon une des exigences précédentes, avec la caractéristique particulière que la zone périphérique (3) comporte au moins partiellement des structures poreuses, en particulier des structures textiles comme zones d'enracinement textiles.

7. Prothèse cornéenne selon une des exigences précédentes, avec la caractéristique particulière que la zone périphérique (3) est, au moins partiellement, colorée, imprimée et/ou de brodée.

8. Prothèse cornéenne selon une des exigences précédentes, avec la caractéristique particulière que la zone périphérique (3) comporte un façonnage afin d'accueillir le matériau de suture.

9. Prothèse cornéenne selon une des exigences précédentes, avec la caractéristique particulière que la surface de la zone périphérique est bioactive par le biais d'une couche, en particulier une couche liée par covalence et biocompatible.

10. Prothèse cornéenne selon une des exigences précédentes, avec la caractéristique particulière que la surface bioactive de la zone périphérique (1), en particulier, le cas échéant, son revêtement, comporte des molécules d'ancrage composées d'au moins une liaison chimique par covalence avec la zone périphérique (3) avec au moins un groupe fonctionnel.

11. Prothèse cornéenne selon l'exigence 10, avec la caractéristique particulière que la surface bioactive de la zone périphérique, en particulier, le cas échéant, son revêtement, comporte des biomolécules avec un groupe fonctionnel, telles que les biomolécules sont reliées par au moins un de leurs groupes fonctionnels avec des groupes fonctionnels des molécules d'ancrage.

12. Prothèse cornéenne selon l'exigence 11, avec la caractéristique particulière que les biomolécules présentent au moins un centre bioactive pour l'accrochage de cellules.

13. Prothèse cornéenne selon les exigences 11 ou 12, avec la caractéristique particulière que le revêtement de la zone périphérique comporte des molécules espaceurs avec des groupes fonctionnels, telles que les molécules espaceurs sont placées entre les molécules d'ancrage et les biomolécules et que leurs groupes fonctionnels soient liés par covalence aux groupes fonctionnels des molécules d'ancrage et des biomolécules.

14. Prothèse cornéenne selon une des exigences 10 à 13, avec la caractéristique particulière que chaînes de poly(acide acrylique) copolymérisées greffées sont prévus comme molécules d'ancrages.

15. Prothèse cornéenne selon une des exigences 10 à 14, avec la caractéristique particulière que des groupes de carboxyle sont prévus comme groupe(s) fonctionnel(s) des molécules d'ancrage.

16. Prothèse cornéenne selon une des exigences 11 à 15, avec la caractéristique particulière que des groupes amino sont prévus comme groupe(s) fonctionnel(s) des biomolécules.

17. Prothèse cornéenne selon une des exigences 11 à 16, avec la caractéristique particulière que des protéines favorisant l'adhésion de cellules et/ou des fragments de ces protéines sont prévus comme biomolécules.

18. Prothèse cornéenne selon une des exigences 11 à 17, avec la caractéristique particulière que des facteurs de croissance, en particulier le "Platelet Derived Growth Factor" (PDGF) sont prévus comme biomolécules.

19. Prothèse cornéenne selon une des exigences 11 à 18, avec la caractéristique particulière que des peptides de taille réduite comportant des domaines liant les cellules, en particulier des peptides contenant du RGD avec des séquences peptidiques entre 3 et 40 acides aminés sont prévus comme biomolécules.

20. Prothèse cornéenne selon une des exigences précédentes, avec la caractéristique particulière que la surface bioactive de la zone périphérique (3), en particulier, le cas échéant, son revêtement, comporte des couches d'activateurs pour la guérison.

21. Prothèse cornéenne selon une des exigences précédentes, avec la caractéristique particulière que la zone périphérique (3), en particulier, le cas échéant, son revêtement, comporte des catalyseurs pour la fixation de cellules.

22. Prothèse cornéenne selon une des exigences 6 à 21, avec la caractéristique particulière que la zone d'enracinement (4) est formée comme un tissu à mailles, un treillis, un tissu, une mousse, une feuille, un rembourrage ou d'autres éléments poreux ou perforés ainsi qu'une combinaison de tels éléments.

23. Prothèse cornéenne selon une des exigences 6 à 22, avec la caractéristique particulière que la zone d'enracinement (4) consiste au moins partiellement de matériaux réabsorbants.

24. Prothèse cornéenne selon une des exigences 6 à 23, avec la caractéristique particulière que la zone d'enracinement (4) est fermement reliée par la zone périphérique restante et/ou directement avec le centre optique (2).

25. Prothèse cornéenne selon une des exigences 6 à 24, avec la caractéristique particulière que la zone d'enracinement (4) est reliée à la zone périphérique restante et le centre optique (2) ou directement avec le centre optique (2) par affusion, fusion, agglutination, suture, soudure et/ou par copolymérisation.

26. Prothèse cornéenne selon une des exigences 6 à 25, avec la caractéristique particulière que la couche antiadhésive pour le centre optique (2) comporte de l'héparin, sulfate d'héparine et/ou leur dérivés.

27. Prothèse cornéenne selon une des exigences précédentes, avec la caractéristique particulière que pour la détermination de la pression intraoculaire des moyens sont prévus pour mesurer la dilatation dans la région de la zone périphérique (3), en particulier dans la zone d'enracinement tissulaire (4).

28. Prothèse cornéenne selon une des exigences 6 à 27, avec la caractéristique particulière qu'un renforcement et/ou un une embase de blindage au diamètre intérieur de la zone d'enracinement (4) est formé.

29. Prothèse cornéenne selon une des exigences 6 à 28, avec la caractéristique particulière qu'un renforcement vers l'intérieur de l'oeil est formé depuis le diamètre extérieur de la zone d'enracinement (4).

30. Prothèse cornéenne selon une des exigences 6 à 29, avec la caractéristique particulière que la zone d'enracinement (4) présente une porosité ouverte et une distribution de la taille des pores optimisées pour la fixation des cellules.

31. Prothèse cornéenne selon une des exigences précédentes, avec la caractéristique particulière que la surface intérieure et/ou extérieure du centre optique (2) est dotée d'une adaptation dioptrique.

32. Procédé de fabrication d'une couche sur la zone périphérique (3) d'une prothèse cornéenne, liée par covalence, biocompatible et adhérant aux cellules, avec la caractéristique particulière que sur la zone périphérique des molécules d'ancrage sont formées présentant des groupes fonctionnels en créant avec un gaz inerte par un allumage, avec des plasmas à basse pression induits par des micro-ondes, des groupes radicaux qui réagissent après aération avec de l'oxygène pour donner des radicaux péroxy et ensuite des hydropéroxides, lesquels, enfin, forment des radicaux libres par fission thermique et/ou photochimique, par qui les monomères greffés sont copolymérisés.

33. Procédé selon l'exigence 32, avec la caractéristique particulière que un/des groupe(s) fonctionnel(s) de biomolécules sont liés par covalence au(x) groupe(s) fonctionnel(s) des molécules d'ancrages.

34. Procédé selon une des exigences 32 ou 33, avec la caractéristique particulière que des homopolymères excédentaires, non liés par covalence, sont éliminés suite à la copolymérisation greffée.

35. Procédé selon une des exigences 32 à 34, avec la caractéristique particulière que le gaz plasmatique Argon et/ou N_2 est employé.

36. Procédé selon une des exigences 32 à 35, avec la caractéristique particulière que pour la fission photochimique une fission thermique et/ou une fission induite par des rayons UV moyennant un laser excimer est utilisé.

37. Procédé selon une des exigences 32 à 36, avec la caractéristique particulière que des monomères de vinyle sont utilisés comme monomère à greffer, en particulier des acides acryliques (AAc), et/ou des macromonomères, en particulier des methacrylates de polyéthylène glycol (PEGMA), pour la formation d'une couche d'hygrogel hautement hydrophile.

38. Procédé selon l'exigence 37, avec la caractéristique particulière qu'un PEGMA avec une longueur de chaînes de poly(éthylène) entre 4 et 100 pour la formation d'une couche d'hygrogel hautement hydrophile est utilisé.
